# EUROPEAN PATENT APPLICATION

(11) **EP 1 949 846 A1**
(43) Date of publication of application: **30.07.2008**
(21) Application number: 08100713.0
(22) Date of filing: 21.01.2008
(51) Int. Cl.: A61B 1/00, A61B 3/00, A61F 9/00, G02B 5/08, G02B 5/20, G02B 27/00, A61B 1/07

(54) **System and method for the removal of undesired wavelengths from light**

(30) Priority: 23.01.2007 US 626015
(71) Applicant: Alcon, Inc., 6331 Hünenberg (CH)
(72) Inventor: Papac, Michael, Tustin, CA 92780 (US); Buczek, Mark, Oceanside, CA 92054 (US); Smith, Ronald, Irvine, CA 92618 (US); Artsyukhovich, Alex N., Dana Point, CA 92629 (US); Dacquay, Bruno, Irvine, CA 92603 (US)
(74) Representative: Hanna, Peter William Derek

(57) **Abstract**

Embodiments of the present invention provide a system and method for filtering light from a light source (220). More particularly, embodiments of the present invention may filter direct and reflected light provided from the light source to a source of illumination (212) by using one or more mirrors (230a,230b) in an optical path. In a specific embodiment, one or more cold mirrors (250) in the optical path are coated such that they are operable to reflect light in the desired wavelengths while transmitting other wavelengths of light. Thus, as light from a light source is reflected off these cold mirrors undesired wavelengths of light may be substantially removed from the light before it is utilized to illuminate an area. In other embodiments, one or more hot mirrors (260) may also be provided in the optical path, where these hot mirrors are coated such that they are operable to transmit the desired wavelengths of light and reflect undesired wavelengths.

## Description

### Technical Field Of The Invention

The present invention relates to sources of illumination. More particularly, embodiments of the present invention relate to the removal of undesired wavelengths from light. Even more particularly, embodiments of the present invention relate to systems and methods for removing undesired wavelengths from light using mirrors.

### Background

The human eye can suffer a number of maladies causing mild deterioration to complete loss of vision. While contact lenses and eyeglasses can compensate for some ailments, ophthalmic surgery is required for others. Generally, ophthalmic surgery is classified into posterior segment procedures, such as vitreoretinal surgery, and anterior segment procedures, such as cataract surgery. More recently, combined anterior and posterior segment procedures have been developed.

The surgical instrumentation used for ophthalmic surgery can be specialized for anterior segment procedures or posterior segment procedures or support both. Such surgical instrumentation can comprise a Vitreoretinal and Cataract microsurgical console. Such a surgical console can provide a variety of functions depending on the surgical procedure and surgical instrumentation. For example, surgical consoles can expedite cataract surgeries (e.g. phacoemulsification procedures) by helping manage irrigation and aspiration flows into and out of a surgical site. Surgical consoles can also provide other functions, as will be known to those having skill in the art.

Clearly seeing an area upon which a surgical procedures is being performed is obviously important. Therefore, microsurgical consoles typically provide a source of illumination, such that the source of illumination can be used to illuminate the area on which a procedure is being performed. These illuminators provide visible light from a light source through, for example, an optic fiber or the like.

In many cases, the light source may generate wavelengths of light which are not useful for illumination (e.g., those other than visible light), such as ultraviolet (UV) or infrared (IR) light. These wavelengths may adversely affect the surgical system by, for example, causing a reduction in the lifetime of lamp electrodes, thermal and UV damage to the system optical components over time, decreased optical performance due to spherical and chromatic aberrations, beam steering, etc. More specifically, the illuminator bulb electrodes, due to absorbing this undesired radiation, will erode more quickly, the optics of the system may heat up significantly leading to reliability issues (i.e. thermal shock and cracking) in the optics as well as their optical coatings, and thermal expansion can induce spherical and chromatic aberrations in the optics of the system, further reducing the performance of the system. This is an undesirable effect because damage to the surgical system optics or their optical coatings can lead to a dangerous amount of light passing through the optics and into the eye, resulting in eye damage. Furthermore, unwanted UV or IR radiation that reaches the eye can potentially cause eye damage.

In past illuminators the problem of reducing out of band emissions was addressed by placing a band pass mirror (also called a "hot mirror") in the optical path to absorb or reflect undesired wavelengths of light. However, no specific hardware or methods were put in place to eliminate undesired radiation at the illuminator optical source or from the optical train upstream of the hot mirror. Thus, during the operation of these prior art systems the hot mirror and the optics upstream of the hot mirror continued to heat to unacceptable levels. Therefore, there is a need for a system and method that can effectively and efficiently reduce or eliminate undesired wavelengths in light provided by a light source.

### Summary Of The Invention

Embodiments of the present invention provide an illumination system and method that can be utilized in conjunction with a surgical console and which are substantially safer, more durable and less expensive to operate than prior art systems and methods for providing illumination. One embodiment of the present invention includes a method for providing light to a source of illumination. The method can comprise the steps of: providing light from a light source wherein the light has two components, one component reflected from a mirror having a coating operable to reflect desired wavelengths of light and transmit undesired wavelengths and a second component provided directly from the light source (e.g. not reflected off the mirror). Other embodiments may provide other mirrors to further reduce the undesired wavelengths in the light provided through the source of illumination. For example, an additional coated mirror may be provided such that the light (e.g. both components) may be filtered using this mirror. One of these mirrors may be a hot mirror coated to reflect the desired wavelengths and transmit the undesired wavelengths.

By removing undesired wavelengths of light, such as those that fall outside the visible spectrum or certain wavelengths of blue-light, embodiments of the present invention provide the advantage that the lifetime and reliability of components within the optical path of an illuminator may be extended by reducing the amount of radiation absorbed by components within the optical path while simultaneously improving the performance of the illuminator by reducing spherical and chromatic aberrations. By reducing or eliminating such problems in the optical path, the safety of illumination systems incorporating an embodiment of the present invention, especially in a surgical context, may be greatly improved.

These, and other, aspects of the invention will be better appreciated and understood when considered in conjunction with the following description and the accompanying drawings. The following description, while indicating various embodiments of the invention and numerous specific details thereof, is given by way of illustration and not of limitation. Many substitutions, modifications, additions or rearrangements may be made within the scope of the invention, and the invention includes all such substitutions, modifications, additions or rearrangements.

### Brief Description Of The Figures

A more complete understanding of the present invention and the advantages thereof may be acquired by referring to the following description, taken in conjunction with the accompanying drawings in which like reference numbers indicate like features and wherein:
FIGURE 1 is a diagrammatic representation of one embodiment of a surgical console in accordance with the embodiments of this invention;
FIGURE 2 is a representation of one embodiment of an illuminator in accordance with the embodiments of this invention;
FIGURE 3 is a graphical representation of the operational ability of embodiments of various coatings; and
FIGURE 4 is a graphical representation of the operational ability of embodiments of various coatings in accordance with this invention.

### Detailed Description

Preferred embodiments of the invention are illustrated in the FIGURES, like numerals being used to refer to like and corresponding parts of the various drawings.

Vitreoretinal and Cataract microsurgical consoles can provide an illuminator. These illuminators provide visible light from a light source through, for example, an optic fiber or the like, and may be used to illuminate an area on which a procedure is being performed. The light source for these illuminators may generate wavelengths of light which are not useful for illumination (e.g., those other than visible light), such as ultraviolet (UV) or infrared (IR) light, or which may cause aphakic hazard weighted irradiance, such as certain wavelengths of blue light. These extraneous wavelengths may adversely affect the surgical system, including causing a reduction in the lifetime of certain components, decreased performance due to spherical and chromatic aberrations or other causes, etc. Damage to the optics or their optical coatings in a surgical setting can allow a dangerous amount of light to pass into the eye, resulting in eye damage. Furthermore, unwanted UV or IR radiation or blue light that reaches the eye can potentially cause eye damage. Thus, it is desirable that an illuminator, whether in a surgical console or a stand-alone device, effectively reduce or eliminate undesired wavelengths in the light that it provides.

Embodiments of the present invention solve this problem by using one or more mirrors in the optical path of an illuminator. In a preferred embodiment, one or more cold mirrors in the optical path are coated such that they are operable to reflect light in the desired wavelengths (e.g. visible wavelengths), while transmitting other wavelengths of light (e.g. IR, UV or selected wavelengths of blue light). Thus, as light from a light source is reflected off these cold mirrors, undesired wavelengths of light may be substantially removed from the light before it is utilized to illuminate an area. In other embodiments, one or more hot mirrors mirror may also be provided in the optical path, wherein these hot mirrors are coated such that they are operable to transmit the desired wavelengths of light and reflect or absorb undesired wavelengths. Thus, by transmitting the light through these hot mirrors additional undesired wavelengths may be removed from the light (relative to utilizing no mirrors, or cold mirrors alone) before the light is used to illuminate an area.

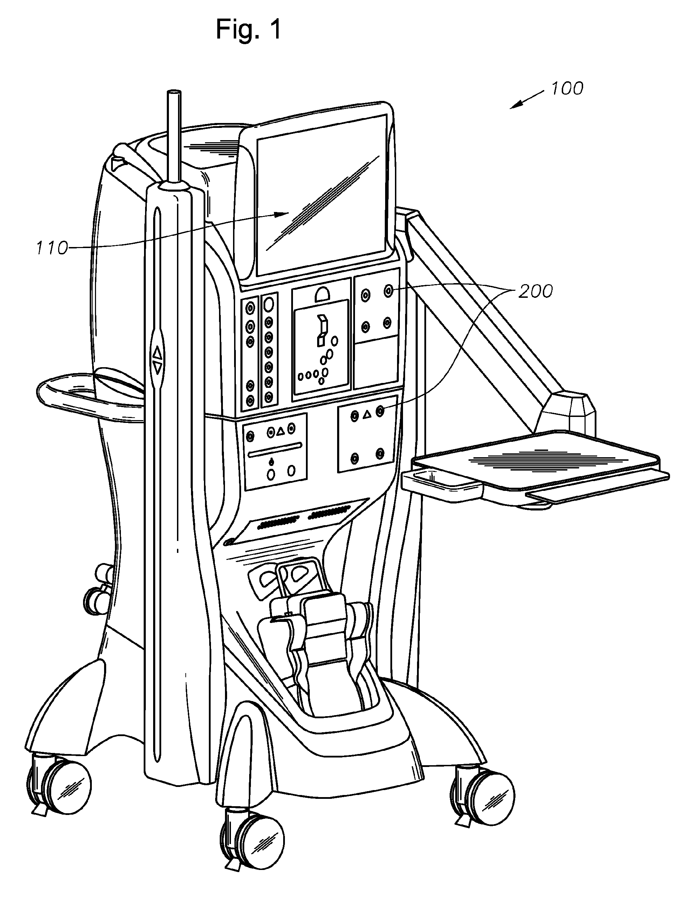

FIGURE 1 is a diagrammatic representation of one embodiment of an ophthalmic surgical console 100. Surgical console 100 can include a swivel monitor 110 that has a touch screen. Swivel monitor 110 can be positioned in a variety of orientations for whoever needs to see the touch screen. Swivel monitor 110 can swing from side to side, as well as rotate and tilt. The touch screen may provide a graphical user interface ("GUI") that allows a user to interact with console 100.

Surgical console 100 also includes a connection panel used to connect various tools and consumables to surgical console 100. The connection panel can include, for example, a coagulation connector, balanced salt solution receiver, connectors for various hand pieces and a fluid management system ("FMS") or cassette receiver. Surgical console 100 can also include a variety of user friendly features, such as a foot pedal control (e.g., stored behind a panel of the console) and other features. One of these features may be one or more illuminators 200 to provide visible (or other wavelengths) of light to an area undergoing examination or surgical procedure.

Surgical console 100 is provided by way of example and embodiments of the present invention can be implemented with a variety of surgical systems. Example surgical systems in which various embodiments of the present invention can be used include, for example, the Constellation® Surgical Console, Series 2000® Legacy® cataract surgical system, the Accurus® 400VS surgical system, and the Infiniti™ Vision System surgical system available from Alcon Laboratories Inc. of Fort Worth, Texas. While embodiments of the invention may be discussed with reference to such surgical consoles, it will be apparent that embodiments of the present invention can be implemented in other suitable surgical systems or, in general, in any system where it may be desirable to provide illumination.

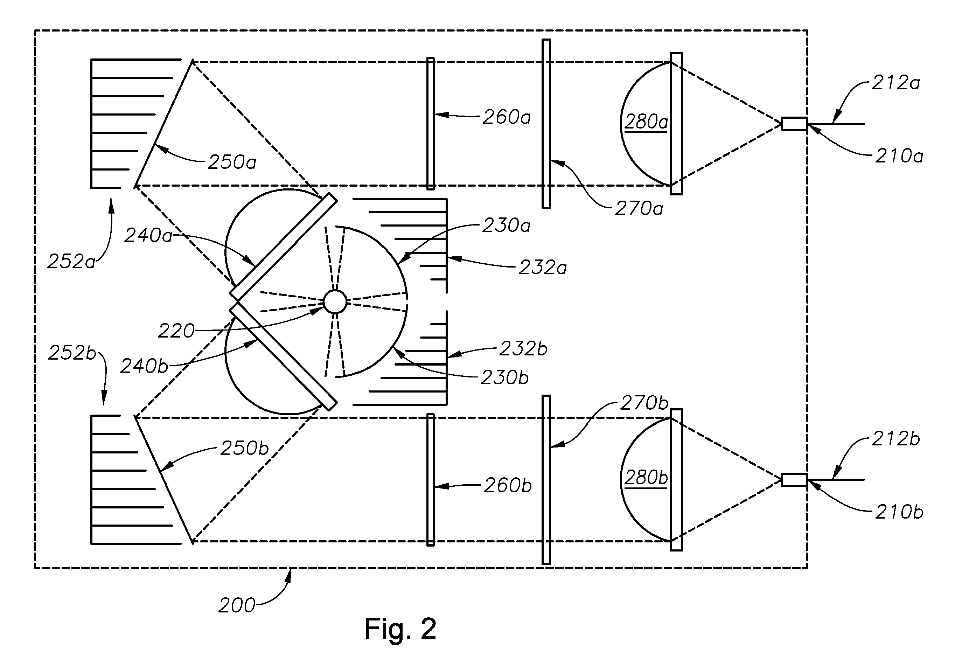

As discussed above, surgical console 100 may provide illuminators 200. FIGURE 2 shows in more detail an embodiment of an illuminator 200. As shown in FIGURE 2, illuminator 200 is a two port illuminator, having two ports 210a and 210b where light from light source 220 is focused onto the proximal end of an illumination delivery device (source of illumination), such as optic fiber 212, for delivery to its eventual destination (e.g. a location at the distal end of optic fiber 212). More particularly, with respect to the optical path corresponding to port 210a, light from light source 220 reflected from spherical mirror 230a and light directly (meaning, in this case, that this light has not been reflected from spherical mirror 230a) from light source 220 is passed through collimating lens 240a, after which the light is reflected from cold mirror 250a (which may be at an angle of approximately 22.5 degrees), transmitted through hot mirror 260a and passed through attenuator 270a before being condensed onto optic fiber 212a by condensing lens 280a. Note that the optical path corresponding to port 210b is substantially similar to that described with respect to port 210a and descriptions applicable to the optical path corresponding to port 210a may, in this particular embodiment, be similarly applied to the optical path corresponding to port 210b.

As mentioned above, it may be desirable to remove undesired wavelengths from light delivered to optic fiber 212a, especially those wavelengths of light that fall substantially within the UV and IR spectrums. To achieve this, in one embodiment of the invention, light from light source 220 is filtered by selecting appropriate glass material or optical coatings for components for one or more components within the optical path of the light from light source 220 such that undesired wavelengths are removed from the light before it is condensed onto optic fiber 212.

More specifically, in one embodiment, specific coatings may be placed on one or more components within the optical path based upon the component's position or function within the optical path. Dichroic coatings are an effective way of separating light in one wavelength band from light in another band by transmitting one wavelength and reflecting another. These dichroic coatings may comprise multiple layers of one or more materials chosen based on their material properties (e.g. refractive indices) and the properties of the substrate material (e.g. glass). The thickness or order in which these coatings are deposited on the optical component may be varied in order to achieve an effect on the transmittance or reflectance of the optical component. Thus, a dichroic coating operable to remove IR and UV may be applied to one or more reflective optics within the optical path of an illuminator while an absorptive/dichroic coating may be applied to the transmittive optics within the optical path.

The use of such coatings may be better elucidated with reference to illuminator 200 depicted in FIGURE 2. In one embodiment, spherical mirror 230 and cold mirror 250 may comprise a glass substrate coated with a dichroic coating operable to transmit or absorb IR and UV wavelengths of light while reflecting visible wavelengths of light (though each of spherical mirrors 230 and cold mirror 250 may comprise different or unique substrates and coatings), while hot mirror 260 may have a glass substrate coated with a dichroic/absorptive coating to reflect or absorb UV and IR wavelengths and transmit visible wavelengths.

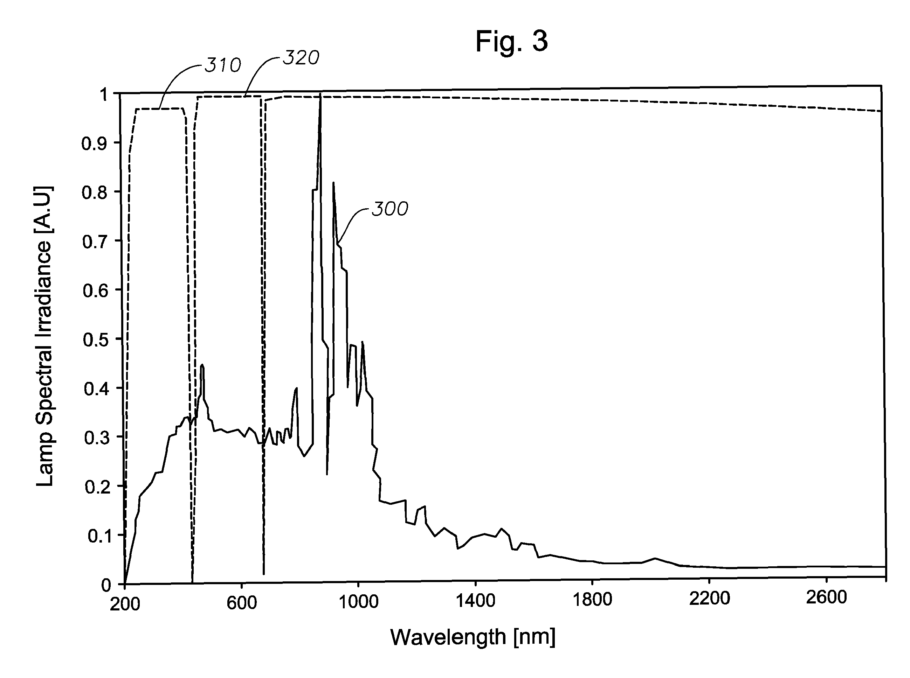

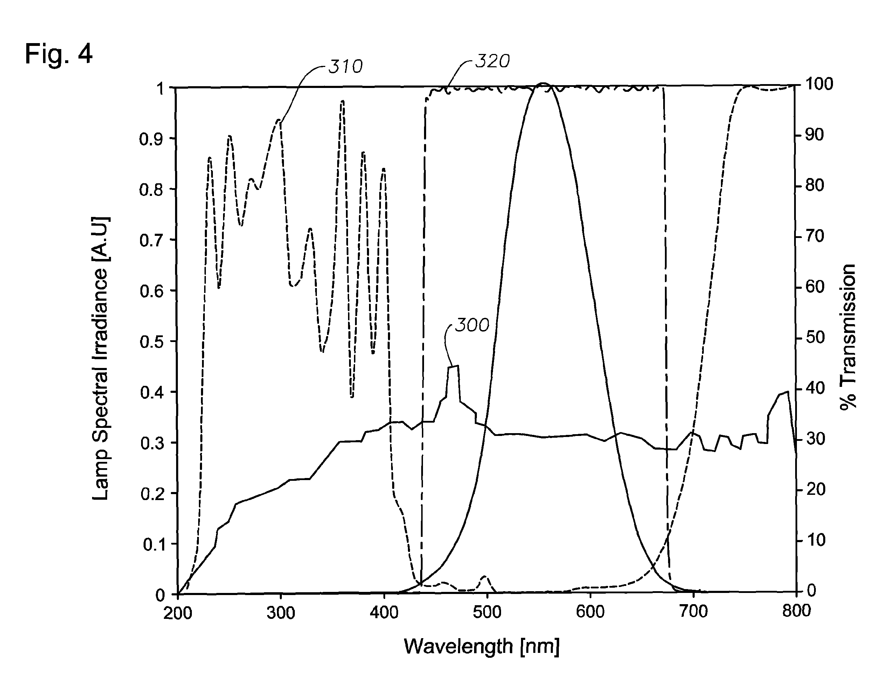

The respective properties of these coatings may be better understood with reference to the graphs of FIGURES 3 and 4 which depict the properties of embodiments of the respective coatings of the optical components within the optical path of illuminator 200. With reference specifically to FIGURE 3, line 300 represents the spectra of light source 220, which in one embodiment may be a substantially ozone free Xenon short arc lamp such as that made by Osram. Notice that this light source has significant emissions outside the spectrum of visible wavelengths (400-700nm). Line 310 represents the transmittive performance of the coating applied to both spherical mirror 230 and cold mirror 250, while line 320 represents the transmittive performance of the coating of hot mirror 260. Notice that the coating applied to spherical mirror 230 and cold mirror 250 (represented by line 310) results in the effective transmission of wavelengths of light substantially outside the visible spectrum while the coating applied to hot mirror 260 (represented by line 320) results in the transmission of light substantially within the visible spectrum.

The efficacy of these respective coatings may be illustrated in more detail with reference to FIGURE 4 which depicts the properties of the embodiments of the respective coatings on a greater scale. Again, notice how the coating applied to spherical mirror 230 and cold mirror 250 (represented by line 310) results in the effective transmission of wavelengths of light substantially outside the visible spectrum while the coating applied to hot mirror 260 (represented by line 320) results in the transmission of light substantially within the visible spectrum.

The effect of the various coated components within the optical path may be explained with reference back to FIGURE 2. Referring to the optical path corresponding with port 210a, light directly from light source 220 and light reflected from spherical mirror 230a is passed through collimating lens 240a. However, as spherical mirror 230a may be coated with a dichroic coating operable to transmit (or absorb) IR and UV wavelengths of light while reflecting visible wavelengths of light as mentioned above, the light may be filtered using spherical mirror 230a, and thus light reflected from spherical mirror 230a may comprise light substantially within the visible wavelengths while out of band or undesired wavelengths (e.g. light not within the visible wavelengths) may be absorbed or collected in out-of-band radiation dump 232a. Thus, light passing through collimating lens 240a may comprise a first component of light reflected from spherical mirror 230a from which wavelengths of light outside of the visible spectrum have been substantially removed (e.g. filtered) and a second component of light directly from light source 220 which still comprises wavelengths outside the visible spectrum. After passing through collimating lens 240a, then, the light is reflected from cold mirror 250a.

As cold mirror 250a may similarly be coated with a dichroic coating operable to transmit (or absorb) IR and UV wavelengths of light while reflecting visible wavelengths of light, the light may be filtered using cold mirror 250a and light reflected from cold mirror 250a may comprise light substantially within the visible wavelengths while remaining out of band wavelengths may be absorbed or collected in out of band radiation dump 252a. The light reflected from cold mirror 250a may then be passed through hot mirror 260a, which is coated with a dichroic/absorptive coating operable to reflect (or absorb) UV and IR wavelengths and transmit visible wavelengths. Thus, by filtering the light using hot mirror 260a, any residual wavelengths of light not within the visible spectrum are reflected by hot mirror 260a and absorbed or collected in out of band radiation dump 252a while visible wavelengths are transmitted through hot mirror 260a and attenuator 270 before being condensed onto optical fiber 212a by condensing lens 280a. In one embodiment, hot mirror 260a may additionally be tuned to meet any desired aphakic properties of illuminator 200.

Thus, by using various coated optical components within an optical path to remove undesired wavelengths of light a number of advantages may be achieved. Namely, the use of a dichroic spherical mirror may prevent undesired wavelengths from re-imaging onto the bulb electrodes and decrease the amount of out-of band light that could be potentially absorbed into optical components in the optical path downstream. By reducing the amount of out-of-band light at various points in the optical path the operating temperature of any hot mirrors within the optical path may be reduced, reducing the likelihood of component failure or degradation. Similarly, by absorbing undesired wavelengths of light transmitted or reflected by the various mirrors, the amount of radiation absorbed by other components within the system may be reduced, reducing the heating or other stresses placed on these components. Furthermore, by applying different coatings based upon the type of light source utilized in a particular embodiment of illuminator, various specifications (e.g., hazard specifications or the like) may be obtained.

Although the present invention has been described in detail herein with reference to the illustrated embodiments, it should be understood that the description is by way of example only and is not to be construed in a limiting sense. It is to be further understood, therefore, that numerous changes in the details of the embodiment of this invention and additional embodiments of this invention will be apparent, and may be made by, persons of ordinary skill in the art having reference to this description. For example, thought embodiments of the invention have been described in conjunction with a two port illuminator having a spherical mirror, a cold mirror and a hot mirror it will be understood that embodiments of the present invention may apply equally well to illuminators with more or fewer ports or more or fewer spherical, cold or hot mirrors. It is contemplated that all such changes and additional embodiments are within scope of the invention as claimed below.

## Claims

1. A method for removing undesired wavelengths of light, comprising:
providing light from a light source (220) to a source of illumination (212), the light having a first component and a second component, the first component provided directly from the light source; and
filtering the second component of light with a first mirror (230), wherein the first mirror has a coating operable to reject light of undesired wavelengths.

2. The method of claim 1, wherein the coating of the first mirror is operable to reflect light in desired wavelengths and transmit light of undesired wavelengths.

3. The method of claim 2, wherein the first mirror (230) is a spherical mirror

4. The method of claim 3, wherein the coating of the first mirror (230) is a dichroic coating.

5. The method of claim 4, further comprising absorbing the undesired wavelengths of light transmitted by the first mirror (230) in a first radiation dump (232).

6. The method of claim 1, further comprising filtering the light using a second mirror (250), wherein the second mirror has a coating operable to reject light of the undesired wavelengths.

7. The method of claim 6, wherein the coating of the second mirror (250) is operable to reflect light in the desired wavelengths and transmit light of the undesired wavelengths.

8. The method of claim 7, wherein the coating of the second mirror (250) is a dichroic coating.

9. The method of claim 8, wherein the second mirror (250) is at an angle of approximately 22.5 degrees.

10. The method of claim 9, further comprising absorbing the undesired wavelengths of light transmitted by the second mirror (250) in a second radiation dump (252).

11. The method of claim 6, further comprising filtering the light with third mirror (260), the third mirror having a coating operable to reject light of the undesired wavelengths.

12. The method of claim 11, wherein the coating of the third mirror (260) is operable to reflect light in the undesired wavelengths and transmit light in the desired wavelengths.

13. The method of claim12, wherein the coating of the third mirror (260) is a dichroic and absorptive coating.

14. The method of claim 13, further comprising absorbing the undesired wavelengths of light reflected by the third mirror (260) in the second radiation dump (252).

15. The method of claim 11, further comprising passing the light reflected from the first mirror (230) through a collimating lens (240) before filtering the light with the second mirror (250).

16. The method of claim 15, wherein the light is filtered with the second mirror (250) before it is filtered with the third mirror (260).

17. The method of claim 16, further comprising condensing the light onto the source of illumination (212) using a condensing lens (280).

18. A system, comprising:
a light source (220);
a first mirror (230) having a coating operable to reject light of undesired wavelengths; and
a source of illumination (212), wherein source of illumination is operable to provide light from the light source, the light having a first component and a second component, the first component provided directly from the light source and the second component filtered with the first mirror.

19. The system of claim 18, wherein the coating of the first mirror (230) is operable to reflect light in desired wavelengths and transmit light of undesired wavelengths.

20. The system of claim 19, wherein the first mirror (230) is a spherical mirror.

21. The system of claim 20, wherein the coating of the first mirror (230) is a dichroic coating.

22. The system of claim 21, further comprising a first radiation dump (232) operable to absorb the undesired wavelengths of light transmitted by the first mirror (230).

23. The system of claim 18, further comprising a second mirror (250) having a coating operable to reject light of the undesired wavelengths wherein the light is filtered using the second mirror.

24. The system of claim 23, wherein the coating of the second mirror (250) is operable to reflect light in the desired wavelengths and transmit light of the undesired wavelengths wherein the light is filtered using the second mirror.

25. The system of claim 24, wherein the coating of the second mirror (250) is a dichroic coating.

26. The system of claim 25, wherein the second mirror (250) is at an angle of approximately 22.5 degrees.

27. The system of claim 26, further comprising a second radiation dump (252) operable to absorb the undesired wavelengths of light transmitted by the second mirror (250).

28. The system of claim 23, further comprising a third mirror (260) having a coating operable to reject light of the undesired wavelengths, wherein the light is filtered by the third mirror.

29. The system of claim 28, wherein the coating of the third mirror (260) is operable to reflect light in the undesired wavelengths and transmit light in the desired wavelengths.

30. The system of claim 29, wherein the coating of the third mirror (260) is a dichroic and absorptive coating.

31. The system of claim 30, wherein the undesired wavelengths of light reflected by the third mirror (260) are absorbed in the second radiation dump (252).

32. The system of claim 28, further comprising a collimating lens, wherein the light is passed through the collimating lens (240) before it is filtered with the second mirror (250).

33. The system of claim 32, wherein the light is filtered with the second mirror (250) before it is filtered with the third mirror (260).

34. The system of claim 33, further comprising a condensing lens (280) operable to condense the light onto the source of illumination (212).

35. An illuminator, comprising:
a light source (220);
a first optical path, the first optical path, comprising
a first source of illumination (212a),
a first mirror (230a) wherein the first mirror is spherical and has a coating operable to reflect light of visible wavelengths and transmit or absorb light of IR and UV wavelengths,
a second mirror (250a) having a coating operable to reflect light of visible wavelengths and transmit or absorb light of IR and UV wavelengths,
a third mirror (260a) having a coating operable to reflect or absorb light in the IR and UV wavelengths and transmit light of visible wavelengths, wherein the source of illumination is operable to provide light from the light source, the light having a first component and a second component, the first component provided directly from the light source and the second component filtered with the first spherical mirror before the light is filtered using the second mirror and the third mirror,
a first radiation dump (232a) operable to absorb the light transmitted by the first spherical mirror, and
a second radiation dump (252a) operable to absorb the light transmitted by the second mirror and reflected by the third mirror; and
a second optical path, the second optical path, comprising
a second source of illumination (212b),
a fourth mirror (230b) wherein the fourth mirror is spherical and has a coating operable to reflect light in the IR and UV wavelengths and transmit light of visible wavelengths,
a fifth mirror (250b) having a coating operable to reflect light in the IR and UV wavelengths and transmit light of visible wavelengths,
a sixth mirror (260b) having a coating operable to reflect light in the IR and UV wavelengths and transmit light of visible wavelengths, wherein the second source of illumination is operable to provide light from the light source, the light having a first component and a second component, the first component provided directly from the light source and the second component filtered with the fourth spherical mirror before the light is filtered using the fifth mirror and the sixth mirror,
a third radiation dump (232b) operable to absorb the light transmitted by the fourth spherical mirror, and
a fourth radiation dump (252b) operable to absorb the light transmitted by the fifth mirror and reflected by the sixth mirror.
